# EUROPEAN PATENT SPECIFICATION

(11) **EP 4 577 259 B1**
(45) Date of publication and mention of the grant of the patent: **06.05.2026**
(21) Application number: 23768452.7
(22) Date of filing: 21.08.2023
(51) Int. Cl.: A61M 1/00

(54) **VACUUM PUMP**
VAKUUMPUMPE
POMPE À VIDE

(30) Priority: 22.08.2022 ZA 202209359
(43) Date of publication of application: 02.07.2025
(73) Proprietor: Shahim, Clinton Frederick, 2055 Johannesburg (ZA)
(72) Inventor: SCHMITT, Karl-Heinz, 2055 Johannesburg (ZA); VISSER, Rudi, 2055 Johannesburg (ZA); SHAHIM, Clinton Frederick, 2055 Johannesburg (ZA)
(74) Representative: Patentanwälte Ruff, Wilhelm, Beier, Dauster & Partner mbB
(86) International application number: PCT/ZA2023/050049
(87) International publication number: WO 2024/044796

(56) References cited:
- WO-A1-2021/024164
- US-A1- 2007 032 763
- US-A1- 2020 376 175
- US-A1- 2021 121 609

## Description

### BACKGROUND OF THE INVENTION

This invention relates generally to negative pressure wound therapy and more particularly to the control of vacuum pressure (referred to as negative pressure) at a site which is subject to this form of therapeutic treatment.

Negative pressure describes a pressure which is below normal atmospheric pressure and which is established at a wound site for therapeutic treatment purposes. The prior art is replete with devices used to establish negative pressure. Typically some form of pump, which may be chemical, electrical or mechanical, is used to lower the pressure at a wound site which is covered by a dressing. The benefits which are associated with this treatment regime are listed in many documents and are not repeated here.

A device which is used on a patient to promote negative pressure therapeutic treatment should, to the extent which is possible, not adversely affect the patient. Ideally, the device should be small, light in weight, and not operate at a noise level which is uncomfortable to the patient. If the device is to be of a portable nature it should for example be battery-driven. In that case power consumption should be minimised so that battery lifetime is extended. Other factors which affect patient acceptability or tolerance of the device include ease of use and reliable and easy to understand indications of operation.

If the treatment process is such that exudate is to be drawn from a wound site then provision must be made for directing the exudate into a container which can easily be connected to a negative pressure device. The container, once filled with exudate, must be disposed of and replacement thereof, with a fresh container, should be effected readily and hygienically.

US 2021/121609 describes a therapy device which establishes a negative pressure at a wound site under the control of a processor. The device uses a constant voltage source which is connected to an air pump motor. No account is taken of the effect of voltage variations in the constant voltage source nor of the effect of the operating temperature of the air pump motor on the value of the negative pressure which is established. A similar observation is made in respect of the disclosure in US2020/376175 wherein an air pump motor is stopped when the amplitude of a current supplied to the motor is at a prescribed level.

US2007/032763 and WO2021/024164 also link vacuum level at a wound site to the current drawn by an air pump motor.

The aforementioned disclosures fail to recognise issues which can adversely affected the relationship between the current which is drawn by a motor and the negative pressure level which is established by an air pump driven by the motor.

An object of the present invention is to provide a method of and an apparatus for controlling negative pressure at a wound site which, at least to a substantial extent, meet the aforementioned requirements and which, according to requirement, can be used with, or without, an exudate-collecting container. Additionally the present invention seeks to obtain a more accurate relationship between the amplitude of current supplied to an air pump motor and a negative pressure which is established by the air pump.

### SUMMARY OF THE INVENTION

The invention is defined in the appended claims.

In a negative pressure wound therapy system, a non-claimed embodiment of the disclosure provides a method of controlling a negative pressure level prevailing at a wound site which includes the steps of (a) using an electrical power supply to power an electric motor which drives an air pump, (b) using the pump to pump air from a region which encloses the wound site, (c) monitoring the amplitude of the current which is supplied by the electrical power supply to the electric motor while the pump is operable, (d) interrupting the power supply to the electric motor when the amplitude of the current reaches a predetermined value, and (e), after a time period, repeating steps (a), (b), (c) and (d).

The electrical power supply may have a variable voltage but in a preferred embodiment the electrical power supply is configured to function as a constant voltage source. Techniques for establishing a constant voltage source are known in the art and are not repeated here.

The pump may be of a type which is referred to as a micro-vacuum pump. By monitoring the amplitude of the current which is supplied to the electric motor, an indication is obtained of the vacuum level at the wound site i.e. of the negative pressure at the wound site. In this respect use may be made if desired of a look-up table which is established beforehand. A look-up table of this kind reflects the relationship between the current which is supplied to the electric motor when the pump is working at a known voltage, and the negative pressure level prevailing at the wound site. It has been established through experimentation that as the negative pressure level increases (i.e. as the vacuum level increases), the electric motor is put under increasing strain and, as a consequence, the current which is drawn by the electric motor is increased. The increase in current is thus dependent on the vacuum level at the wound site and, through experimentation and testing, a look-up table or some other reference system can be established which records the relationship of current to negative pressure level, to an acceptable degree of accuracy.

The look-up table is preferably embodied in software or firmware which is accessed by a controller or processor which is responsive to the amplitude of the current supplied to the electric motor.

The current which is drawn by the electric motor at a particular operating load increases as the temperature of the motor decreases. To correct for this effect the invention includes the steps of monitoring the temperature of the motor, producing a corrective value which is indicative of the change in the current supplied to the motor due to the temperature variation and then using the corrective value to correct the predetermined value referred to in step (d). This process allows the method of the invention to be implemented in a manner which, in substance, is independent of temperature variations.

In step (d) the power supplied to the electric motor is interrupted. As the pump is then not operable and even though the site may be covered by a wound dressing the pressure which is prevailing at the wound site will increase (i.e. the negative pressure decreases) at a rate which is variable and which depends on a variety of factors such as air leakage, the status of the wound dressing and other factors. It has been established through multiple tests that in a typical case, when the pump is not operating the negative pressure level at the wound site will decrease (the vacuum level decreases), under average conditions, to a threshold value within a defined period. Without being limiting the duration of the defined period may vary from 1 to 60 minutes. In one form of the invention at the end of that defined period the connection of the electrical power supply to the electric motor is restored and steps (a), (b), (c) and (d) are repeated so that the negative pressure level at the wound site is again increased (vacuum level is increased).

In a different form of the invention the electrical power supply is reconnected to the electric motor for a brief period which is of sufficient duration to enable a measure of the current, which is then supplied to the electric motor, to be measured. From this current measurement a determination is made of the level of the negative pressure prevailing at the wound site. This process can be repeated at intervals and once it is determined that the negative pressure level prevailing at the wound site has dropped to a lower threshold value the power supply to the pump motor is restored to increase the vacuum level. Otherwise stated the negative pressure level is sampled, at intervals, during a period in which the motor is not continuously operated, by actuating the motor for a time period which is short but of sufficient duration to enable an accurate current measurement to be made. The aforementioned steps are then repeated.

Thus it falls within the scope of the invention for the power supply to the electric motor to be restored after a predetermined time interval, or for the power supply to the electric motor to be restored when a threshold value of the negative pressure level prevailing at the wound site is reached (the vacuum level is inadequate).

A variation in the first approach is to actuate the motor at the end of the predetermined time interval and then to keep the motor in operation if the negative pressure level is less than an acceptable value. If the negative pressure level is above a lowest acceptable value then the operation of the pump is stopped for a brief period whereafter the process is repeated. This approach is adopted to conserve the operating lifetime of the electrical power supply.

In each form of the invention the method may include the step of compensating for variations in the current supplied to the motor which are due to temperature effects.

The method may include the step of obtaining a measure of the no-load current of the motor i.e. when the pump is not operable. This measure is taken into account when relating the current which is drawn by the motor to the prevailing negative pressure level at the wound site. Conveniently this is done by subtracting the measure of the no-load current from the measure of the actual current which at the time, is supplied to the motor. In this way the magnitude of the current required for the motor and pump to operate at the prevailing negative pressure level, is more accurately assessed.

The method may include the further step of monitoring the amplitude of the current which is supplied to the motor as a function of time. The current amplitude is compared to a reference value. If the amplitude of the current at any given time in a cycle of operation is below the reference value, possibly determined through experimentation, for that given time, then it is possible to detect that there is an operative malfunction in that air leakage is taking place either in the equipment which is being used to establish the negative pressure, at the wound site, or at some mechanism e.g. a container which is positioned between and which is connected to such equipment and to the wound site. A possible warning, audible or visual or both, can be generated and corrective action can be taken.

If the amplitude of the current which is supplied to the motor at any given time in a cycle of operation is above a reference value for that given time, established via a comparative process then, again, existence of an operative malfunction is evident in that some form of air blockage may have occurred either in the equipment which is used to establish the negative pressure or, possibly, at the wound site, for example that a dressing has become blocked. Again, corrective action can be taken in response to a warning signal.

As referred to herein each "cycle of operation" refers to a time period which commences once the motor is turned on and extends until the motor is turned off.

In a modification of this technique the rate of change of the negative pressure level is monitored to obtain an indication of the efficacy of the method. When the pump is operating, if the rate of change is too slow i.e. below a value which is determined e.g. in a workshop for ideal operating conditions, it is likely that there is an air leak in the system and corrective action is required. If the rate of change is above a norm i.e. too high, then this may be indicative of an air blockage in the system or that a wound dressing, at the wound site, is saturated (blocked).

Another non-claimed embodiment of the disclosure further extends to an apparatus, for use in a negative pressure wound therapy system, for controlling a negative pressure level prevailing at a wound site, the apparatus including an electrical power supply, an electric motor which is powered by the power supply, an air pump which is driven by the electric motor, a connection from the pump which is configured, during operation of the pump, to draw air from a region which encloses the wound site, a current monitor which is configured to monitor the amplitude of the current which is supplied by the power supply to the electric motor while the pump is operable, and a processor which, in response to the current monitor, is configured to cause the power supply to the electric motor to be interrupted when the amplitude of the current reaches a predetermined value.

The apparatus may include a temperature sensor which senses the temperature of the motor during operation and which produces a signal which is dependent on the motor temperature and which is applied to the processor. The processor, in turn, in accordance with predetermined criteria, is configured to adjust the monitored amplitude of the current to compensate for the effect on the current which is due to the operating temperature of the motor. The adjusted current value is then indicative of the load on the motor.

The power supply is preferably a constant voltage source. To enable the apparatus to be portable the power supply may make use of one or more batteries. The processor may be configured to monitor the voltage of the batteries and to generate a signal, audible or visual, to indicate to a user that the battery or batteries should be replaced.

The processor may monitor the duration of each period of time for which the motor is operative and produce a sum of these time periods (i.e. accumulate the time periods). When the value of the sum exceeds a predetermined value, which is dependent on one or more factors, the processor may automatically terminate operation of the motor e.g. by permanently interrupting the supply of electric power to the motor. This is done to ensure that usage of the apparatus by a patient (a person who has the wound site) complies with directions which may have been given by medical personnel.

The pump is configured, through suitable mechanical design, to ensure that the maximum negative pressure value (highest degree of vacuum) which can be achieved by means of the pump is restricted. This is a safety feature which ensures that if the pump is inadvertently operated continuously, due for example to a system failure, the vacuum level which is produced by the pump cannot reach a value which could be harmful to a patient. An effective way to achieve this objective is to use an electric motor and a vacuum pump which are not able, even when operating continuously, to produce a vacuum level which is greater than a predetermined maximum negative pressure value.

In one embodiment of the invention a first section, comprising the pump and the electric motor, is separable from a second section which comprises the power supply and the processor. This allows the first section to be disposed of after a defined period of use while the second section comprising the power supply and processor is then made available for reuse.

In a different embodiment the first section and the second section are integral in the sense that they make up a single unit. This embodiment presents an opportunity for the apparatus to be calibrated under production (factory) conditions, with firmware, to take into account the relevant operating parameters of the specific pump and associated motor. This facilitates production and allows for more accurate measurements to be made during use of the apparatus. With the other approach different first sections are usable with the same second section comprising the power supply and processor. The motors and pumps in the first sections, although nominally identical to one another, display slightly different operating parameters, in use, and these differences must be taken into account, in a calibration process, to ensure that accurate measurements are made of negative pressure levels.

Data produced by the apparatus, during use thereof, can be logged by being stored in an internal memory. At time intervals, or upon interrogation by an external signal, such data can be transferred using a Bluetooth facility, an NFC (near field communication) technique or the like, to a smart phone, tablet or computer etc. This allows the data to be made available to a user. The data can be downloaded and reviewed. Following from this various settings of the apparatus may be altered e.g. the procedure time which is the period of time for which the patient would receive treatment with the apparatus can be altered and, possibly, a target value of the negative pressure level which is to be maintained by the apparatus can be adjusted.

Descriptive data may be stored together with the logged data e.g. details of the treatment procedure, information on dressings used at the wound site, the times at which the dressings are changed, and user details.

The apparatus referred to may be contained in a housing which comprises a single section housing or, according to requirement, in at least two housing sections which are connectable to each other. The single section housing is preferred for practical reasons which are referred to hereinafter.

The connection from the pump is configured, during operation of the pump, to draw air from a region which encloses the wound site. That connection could be connected directly to a wound dressing over the wound site. However if the wound site gives rise to exudate then the apparatus should be usable with a container, which preferably is disposable. The connection from the pump is then used to lower the pressure in an interior of the container and the low pressure interior in the container is connected via a suitable tube to the wound dressing. Exudate from the wound dressing then travels through the tube into the container.

The container may include an enclosed volume, generally in the form of a manifold, with a number of passages which connect the interior of the container to an interior of the manifold. The connection from the pump is then used to draw air from an interior of the manifold which, via the passages, ensures that air is drawn from the interior of the container. The passages are preferably located at what in use may be regarded as an upper end of the container.

These passages are small, preferably with a maximum transverse dimension of the order of 0,1mm. Air can pass through a passage of this size but exudate would not normally be able to pass through the passage unless, possibly, all the passages were to be blocked. This mechanical feature helps to prevent spillage of the exudate from the container, if the container is not in a vertical orientation.

The container may comprise a first part, a second part which is engageable with the first part, with a seal at an interface between the parts, and a mechanism, e.g. a handle, which keeps the two parts engaged with each other. The seal may include a plurality of openings which place an interior of the first part in air communication with an interior of the second part. The first part may have a plurality of projections e.g. in the form of pins which extend into respective openings in the seal. This technique comprises a preferred arrangement to produce the aforementioned passages each of which is formed by a gap, with a clearance of about 0,1mm, between opposing surfaces of the respective pin and a rim of the opening.

Conveniently a filter which is used to combat odours is positioned inside the manifold. Thus air which is drawn through the passages, into the manifold and then through the connection from the pump, can thereby be filtered.

Air which is drawn by operation of the air pump, into the housing, escapes to atmosphere through openings in the housing provided for the purpose.

Use is preferably made of a bracket to connect the housing releasably to the container.

A part of the bracket may extend from the first part of the container and the housing may be engageable, for example with a clip action, with that extending part of the bracket.

A strap or hook or any similar formation or member may be fixed to or otherwise extend from the bracket - this facilitates handling of an assembly comprising the housing, within which the apparatus referred to is mounted, the bracket, and the container.

### BRIEF DESCRIPTION OF THE DRAWINGS

The invention is further described by way of example with reference to the accompanying drawings in which:
Figure 1 illustrates in block diagram form apparatus according to the invention which is used to implement the method of the invention,
Figures 2 and 3 are a rear view and a side view respectively of a housing in which the apparatus shown in Figure 1 is located.
Figure 4 is a perspective view of an assembly comprising the housing of Figure 2 in the process of being attached to an exudate container,
Figure 5 is a view from a rear side, on a different scale, of the assembly in Figure 4 once the housing has been fixed to the container,
Figure 6 shows the assembly of Figure 5 in perspective,
Figure 7 is a view from the front of the assembly in Figure 5,
Figure 8 is a side view of the assembly of Figure 5, and
Figure 9 is a view in cross section of the assembly taken on a line 9-9 in Figure 8.

### DESCRIPTION OF PREFERRED EMBODIMENTS

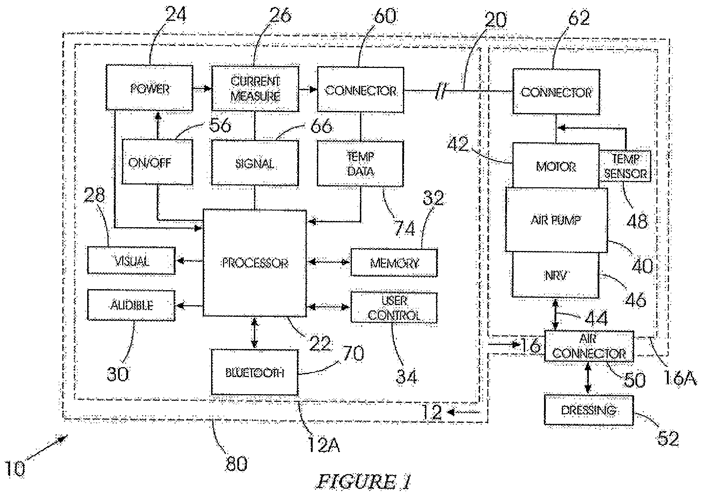

Figure 1 of the accompanying drawings is a block diagram representation of an apparatus 10 according to one form of the invention.

The apparatus 10 consists of a first section 12 which provides a control function, and a second section 16 which is connectable to the first section 12 by means of a cable 20 and which provides a pumping function.

The first section 12 includes a processor 22, a power supply 24, a current monitoring device 26, visual and audible signalling components 28 and 30 respectively, a memory unit 32 which preferably is integrated into the processor 22, and a user control interface 34 which, typically, includes an input device such as a button or switch. The component 28 can be of any suitable form and in one example includes a number of indicating LEDs with signs or labels describing the function of each LED.

The second section 16 includes a diaphragm air pump 40 (also referred to as a micro vacuum pump), an electric motor 42 which actuates the air pump, an air line 44 connected to the air pump 40 via a non-return valve 46, a temperature sensor 48 positioned adjacent the motor 42, and an air connector 50 on the line 44 which is configured to be connected to a dressing 52 in a manner which is known in the art e.g. through the use of a Luerlock connector. The connector 50 provides a suction inlet to the air pump.

In use the dressing 52 is applied to a wound site (not shown) on a patient. The function of the apparatus 10 is to create in a controlled manner a level of negative pressure in a region which is enclosed by the dressing and which is adjacent the wound site. This aspect, which is known in the art, is not described further herein.

The power supply 24 is preferably a constant voltage source. Energy is drawn from replaceable batteries at an appropriate voltage and through the use of electronic circuitry, known in the art, the output voltage which is produced by the batteries is regulated to a constant value.

The processor 22, which is powered by the supply 24, is configured via an output line 56 to turn the power supply on or off, as required in accordance with the operating principles of the invention.

A connector 60 in the first section 12 is coupled to the cable 20. A connector 62 in the second section 16 is coupled to the cable. This arrangement allows power from the supply 24 to pass to the motor 42.

The current monitoring device 26 comprises a current sensing resistor. The voltage drop across the resistor, when current flows through it, is monitored and a corresponding signal 66 is applied to the processor 22.

The visual component 28 can be in any suitable form e.g. a small display screen but conveniently comprises a plurality of LEDs (light emitting diodes) of different colours or in different positions which are chosen so that as the LEDs are selectively operated by the processor 22, in response to operating conditions of the apparatus 10, different output signals are visually made available to a user. In a similar manner audible signals generated by the processor, in response to operating conditions of the apparatus, can be output by the component 30.

The processor 22 is conveniently connected to a Bluetooth module 70 which enables the apparatus 10 to communicate with a smart phone, a tablet or the like (not shown), using a suitable application. The application can be used to check the operating status and operating history of the apparatus. Status conditions include a range of parameters such as run time i.e. the total period of time for which the motor 42 and the air pump 40 have been operated, the operating time left e.g. if the maximum period of treatment has been specified by medical personnel what is the period of time for which treatment must still take place, the level of the power supply 24 i.e. the anticipated time period for which the power supply 24 will still be able to function at an acceptable level, error events and the like.

The processor 22 can be used to monitor all operating conditions, and a log of the resulting information can be stored in the memory unit 32. That data, which can be accessed via the Bluetooth module 70, can record when (time and date) the electric motor 42 was turned on, or off, the period of time for which the motor was on, or off, when (time and date) the operation of the apparatus 10 was interrupted, and any errors that may occur. The data in the memory unit 32 can be transferred, automatically, at regular intervals to a suitably paired receiving device such as a smart phone or tablet and additionally or alternatively, the data can be transferred in response to an interrogating signal from a user.

The data which is transferred to an external device (smart phone or tablet) can be downloaded and viewed by a user. The user thereby will be enabled to alter allowed settings of the apparatus, for example the period of time for which the patient is to receive treatment, and the target level of the negative pressure which should prevail at the wound site during treatment. Descriptive data can also be entered e.g. details of the user, the type of procedure, the dressings which are used, the durations of the time periods for which dressings are used, and the dates and times at which dressings are changed, details of medical personnel associated with the treatment regime, and the like.

The user control 34 preferably is in the form of a tactile pushbutton. Controlled operation of the button can be used to put the apparatus 10 into a working mode, to pause the working mode, and to stop the working mode. The button can also be used in a controlled way to facilitate pairing of the Bluetooth module 70 with a smart phone (not shown) or the like.

The cable 20 is used to supply power to the motor 42 and to the temperature sensor 48. The cable is also used to direct data 74 from the temperature sensor 48 to the processor 22.

In the operation of the apparatus 10 a change in the current which is drawn by the motor and which is due to a variation in the operating state of the air pump must be distinguished from a change in the current drawn by the motor which is attributable to a temperature variation. The electric motor 42 draws more current as its operating temperature drops, for a given pumping state of the air pump 40. Use is made of the temperature sensor 48 to enable corrective data to be generated.

The temperature sensor 48 is based on the use of a resistor with a negative temperature coefficient. Thus the resistance of the resistor decreases as the temperature increases. By measuring the resistance of the resistor the temperature of the motor 42 can be calculated. For this reason the temperature sensor is placed close to the motor as it is this component that is most affected by temperature changes. In this respect use can be made of a thermal transferring compound to place the sensor 48 in good thermal contact with the motor 42, for it is important to measure the temperature of the motor and not the ambient temperature.

The pump 40 is chosen taking into account at least the following factors: current consumption - a low current consumption is needed to obtain an extended operating lifetime from the battery driven power supply 24; a low noise level - this is necessary to minimise disturbance to a patient to whom the wound dressing 52 is applied; size - the pump is as small as possible to limit discomfort to a patient due to weight and size. An additional factor which is of importance and which is taken into account when selecting the pump is to ensure that if the pump stays on indefinitely due to a possible malfunction, the vacuum level which is produced by the continuously operating pump would not become so high as to be harmful to the patient.

The function of the apparatus 10 is to create a negative pressure at an injured area typically a wound which is covered by the dressing 52. The pressure at the wound site is lowered by the pump 40 which is driven by the motor 42, which in turn draws power from the supply 24. It is desirable to maintain the vacuum level (the negative pressure level) at the wound area in a predetermined operating band. To achieve this objective the level of vacuum is controlled by turning the pump 40 on and off in a regulated manner.

As indicated the amplitude of the current which is drawn by the motor, when operating at a constant voltage, is related to the prevailing negative pressure level at the wound site, and the amplitude of the current increases as the negative pressure level at the wound site increases. When the amplitude of the current reaches a predetermined value the motor is turned off. The predetermined maximum amplitude value is directly related to the maximum negative pressure level allowed at the wound site.

When the motor is turned off the pump stops and the negative pressure level at the wound site decreases due to air leakage. After a predetermined time period the motor 42 is turned on and the pump 40 is again operative. At that time if the vacuum level is not low enough i.e. less than a lower threshold value, the pump is kept operative until the required level of negative pressure is again reached. The vacuum level is calculated by measuring the amplitude of the current which is drawn by the motor 42. As the vacuum level increases the motor 42 is put under increasing strain and this increases the amplitude of the current which is drawn by the motor.

The current drawn by the pump motor 42 causes a volt drop over the current sensing resistor embodied in the device 26. The magnitude of the voltage is representative of the amplitude of the current which is dependent on the vacuum level pertaining at the wound site and which is communicated to the air pump 40 through the connector 50 and the line 44.

The motor 42 draws a certain amount of current when operating at no-load. The value of the no-load current (also referred to as the "start-up current") is determined beforehand and stored in the memory unit 32. During operation, at any particular time as may be required for control purposes, the no-load current is subtracted from the current measured by the device 26 to obtain a corrected current reading which is more accurately representative of the value of the current which is supplied in order to achieve a particular negative pressure level at the wound site i.e. the corrected current reading is related more accurately to the previous negative pressure value.

A variation in the temperature of the motor 42 causes the current which is drawn by the motor at a particular operating state of the air pump to change. In general terms the motor draws more current for a particular operating state of the air pump as the motor temperature drops. To address this aspect temperature data 74, produced by the sensor 48, is input to the processor 22. The data is taken into account to determine the value of the current which is supplied to the electric motor 42 and which is attributable only to the operating condition of the air pump 40, at that time i.e. the effect of the operating temperature on the motor is discounted.

When the amplitude of the current supplied to the motor 42 reaches a predetermined value then, by using look-up data in the memory unit 32, the processor can determine that a desired negative pressure level is prevailing at the site of the wound. The motor 42 is then turned off by means of a signal 56 from the processor. After a predetermined time interval, with a duration which can be adjusted according to requirement, the motor 42 is turned on via a signal 56 from the processor 22 and the pump 40 is operated until the amplitude of the current supplied to the motor 42, taking into account the correction factors referred to, is again reached.

In a variation of the invention the electrical supply to the motor is restored for a period which is brief but which is of sufficient duration to enable the amplitude of the current which is drawn by the motor 42 to be measured. This process is continued at spaced time intervals until it is determined by the processor that the negative pressure level at the wound site has reached a lower limit and, in that event, the supply is restored to the motor until such time as a desired operating negative pressure level (an adequate vacuum level) is again established at the wound site. This technique can be adopted to maximise the operating lifetime of the power supply 24.

Relevant data produced during operation is stored in the memory unit 32. This data can be downloaded when required using a suitable interface e.g. an NFC or Bluetooth technique, or any other suitable process, and the data can then be analysed.

The data, once downloaded, can be viewed by a user. Additional data can be added as desired. For example details of medical procedures involved, names of relevant medical personnel, personal details of the user and the like can be entered into the data log. The arrangement may also allow for the variation of control data such as the total time period for which the negative pressure treatment process is to be used, the target value of the negative pressure level to be established at the wound site, and the like.

Operating conditions prevailing in the apparatus 10 can be output to a user via the visual or audible components 28 or 30.

The processor 22 is enabled to monitor the rate of change of the negative pressure at the wound site. Also the amplitude of the current is monitored as a function of time during each cycle of operation i.e. during each period after the pump is turned on until it is turned off. Thus the rate of change of the current, and the magnitude of the current, drawn by the pump motor are monitored. If the negative pressure level increases slowly and, at a given time in a cycle of operation, the amplitude of the current is below a reference value for that given time, as determined by earlier reference data-generating tests, then this is indicative that there may be air leakage in the system. In response a signal is generated via the components 28 and 30 to show that corrective action must be taken. On the other hand if the negative pressure level increases rapidly, above a norm which is established by prior experimentation or if the magnitude of the current at a given time in a cycle of operation is above a reference value for that given time, then this may be indicative that some blockage has occurred in the system, or that the wound dressing may be saturated, or the like - i.e. free air flow is impeded.

In the illustrated embodiment of the apparatus the first section 12 and the second section 16 are units which are mounted in or to respective housings 12A and 16A respectively, shown in dotted outline. The housings 12A and 16A are separable and are interconnected, when required, by means of the arrangement comprising the cable 20 and the connectors 60 and 62. This approach allows the second section 16 to be disposed of after a predetermined period of usage. The first section 12 which normally is not adversely affected by usage, is then available for use with a fresh second section 16. Clearly the batteries in the power supply 24 must be replenished when necessary. In that regard the processor 22 continuously monitors the voltage of the power supply 24 and when this drops to an unacceptable level a signal is output by the processor via the visual component 28 and by the audible component 30.

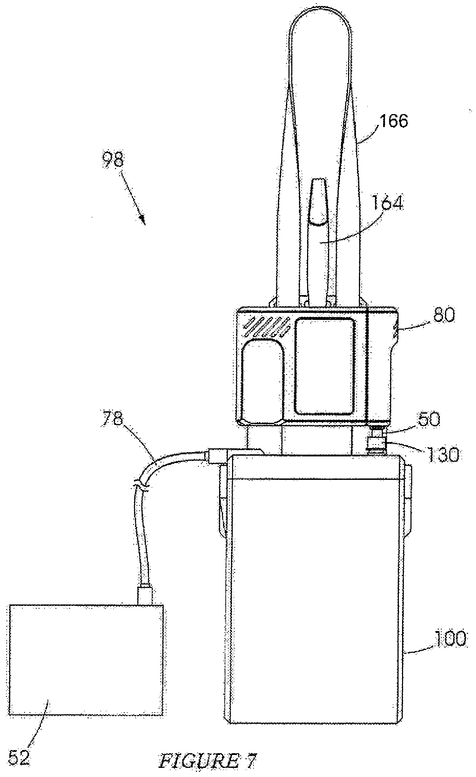

The air connector 50 is positioned on an outer surface of the housing 16A so that it is easily accessible for connection via an appropriate tube 78 to the dressing 52 which is on a wound site of a patient (Figure 7).

The capability in the described embodiment to separate the first section 12 from the second section 16 presents the benefit which has been described. There is however a drawback associated therewith in that it might then be necessary to differentiate between the different combinations of motor and air pump for these combinations, although nominally identical to one another, display different operating parameters in use. For example the speed of operation of a first motor at a particular current level may be slightly different from the speed of operation of a second motor, although nominally identical to the first motor, at the same current level. To correct for such variations, adjustment factors implemented by firmware or software must be used to ensure that a motor current measurement is accurately related to the prevailing operating negative pressure level.

The aforementioned drawback is addressed if the first section 12 and the second section 16 are combined into what may be regarded as an integral or single unit and are mounted in a single housing 80, indicated in dotted outline in Figure 1. The connectors 60 and 62 are dispensed with and direct electrical and physical connections are made to the various components so that these components are all mounted in the single housing 80.

With the integral approach referred to, the apparatus 10 can be factory-calibrated using appropriate firmware, for a specific motor 42 and air pump 40. The result is that production is facilitated and, in use, the current measurements are more accurately related to the negative pressure levels. The operating process of the integral unit i.e. when the first section 12 and the second section 16 are mounted in the single housing 80 is the same as for the arrangement in which the first section 12 and the second section 16 are in separable housings (12A, 16A) and are connected to one another by means of the cable 20.

In either version i.e. if use is made of two housings 12A and 16A, or if use is made of a single housing 80, the arrangement shown in Figure 1 directs air into an interior of a housing. The air escapes from the housing through one of small holes provided for the purpose.

If the wound site is one which gives rise to exudate then the air connector is not directly connected to the dressing 52 but instead is connected to the dressing through the medium of a container into which exudate is discharged, as is described hereinafter.

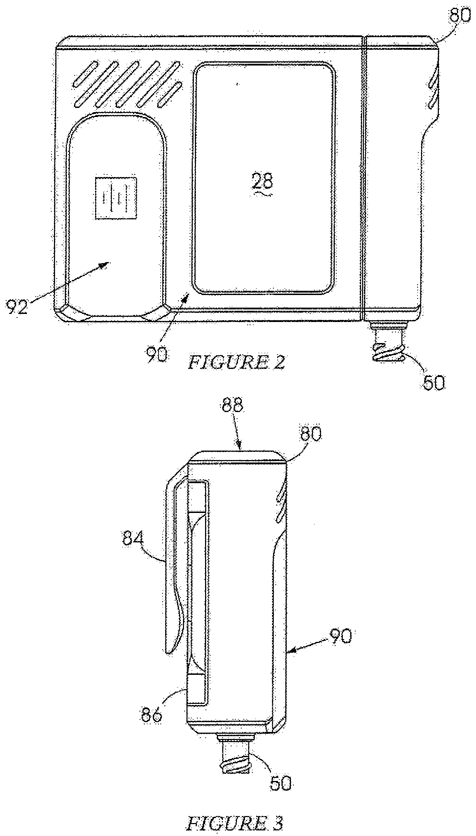

Figure 2 is a rear view of a single housing 80 which contains the apparatus 10. Figure 3 is a side view of the housing. A clip 84 is located on a rear side 86 of the housing. The user control 34 is conveniently positioned on an upper surface 88, on a front side 90 of the housing or at the component 28 which includes LEDs.

The housing 80 has a compartment 92 which contains replaceable batteries which constitute the power supply 24.

The air connector 50, in the form of a small spigot, of conventional design e.g. a Luer connector, projects downwardly from a lower side of the housing.

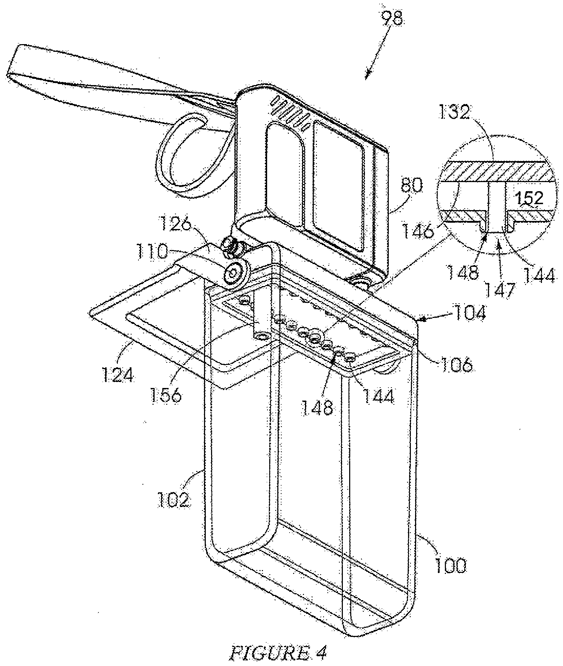

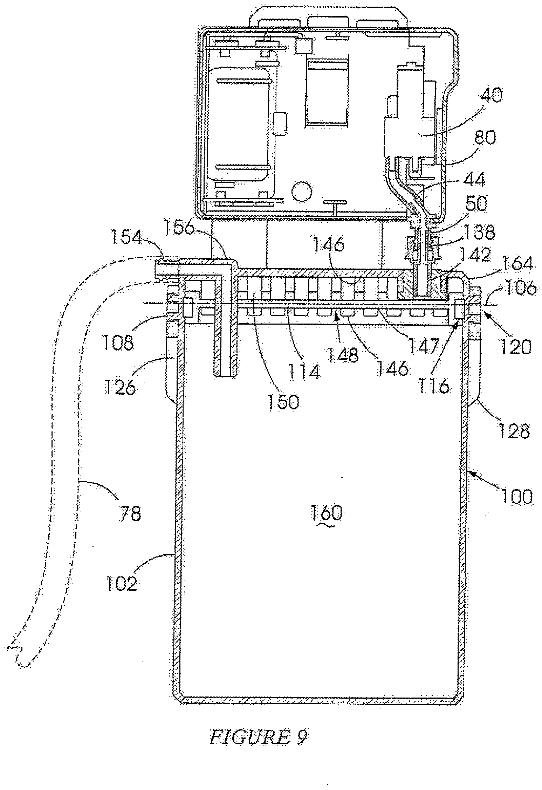

Figure 4 is a perspective view of an assembly 98 comprising the housing 80 attached to a container 100. Internal details of the container 100 are visible in Figure 4 and Figure 9.

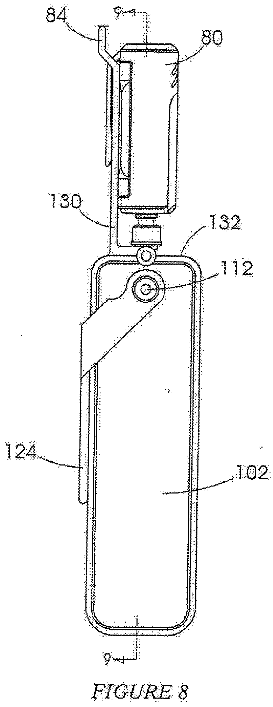

The container 100 comprises a lower part 102 and an upper part 104 which is engageable with the lower part 102. These parts meet on a plane 106. An outer side of each part has a respective half-spigot formation 108 and when the parts are engaged together, see Figure 8, spigots 110 and 112 respectively are formed on opposing sides of the container 100. An insert 114, positioned on the plane 106, has a peripheral channel formation 116 in which is located a resilient seal 120 which acts to seal an interface between the container parts 102 and 104. The two parts are kept engaged with each other by means of a mechanism which comprises a handle 124 which has opposing arms 126 and 128 which are resiliently deformable to a limited extent so that they can engage with a snap action with the spigots 110 and 112 respectively.

A bracket 130 extends from an upper surface 132 of the container part 104. The clip 84 on the housing 80 is releasably engageable with a snap action with the upwardly extending portion of the bracket.

A male Luerlock standpipe fitting 138 is attached to the spigot 50. This is a standard fitting which allows for a push-together connection between the apparatus 10 and the container 100.

The various components are connected together, as shown in Figure 9 to form the assembly 98. The standpipe fitting 138 automatically lines up and engages with a rubber bush 142 which is inserted into a recess in the upper part 104 of the container. This, via the air line 44, provides a leakproof connection between the air pump 40 and the interior of the container 100.

Two rows of pins 144 are moulded onto an inner upper surface 146 of the upper container part 104 and protrude through respective openings 147 in the insert 114, into the lower container part 102. A small passage 148 with a clearance of no more than 0,1mm exists between each pin 144 and a rim of the corresponding opening 146 through which it protrudes (Figure 4).

A manifold 150, defined between the inner upper surface 146 of the upper container part 104 and the insert 114, is filled with a granular graphite filter 152 which neutralises any odours which may be drawn from the exudate in the lower container part 112 through the passages 148 into the manifold 150.

The wound site to be treated is covered with a dressing (not shown) and is connected to a suction port 154 on the container 100 via the flexible hose 78 indicated by means of a dotted line in Figure 9. The suction port 154 is at an end of an elbow 156 which extends into an upper region of the lower container part 102 thereby to place the suction port in air communication with an interior of the container. The hose 78 may include a non-return valve, not shown, to ensure unidirectional flow through the tube.

During operation of the assembly 98 air is drawn from the interior 160 of the lower container part 102 via the vacuum pump 40 into an interior of the housing 80. The air passes through the passages 148 into the manifold 150 and then to the pump. Air discharged into the interior of the housing 80 escapes to atmosphere via small holes (not shown) provided for the purpose.

The handle 124 acts to hold the lower part 102 engaged with the upper part 104 with the insert 114 correctly held in position. The handle may be swivelled away from the container 100 so that the container 100 can be positioned on a flat surface like a bedside or a table. This allows the assembly 98 to be used in an immobile, or mobile, patient environment. When the container 100 is filled with exudate the hose 78 is disconnected from the suction port 154 which is then closed with a lock plug (not shown). The housing 80 is disengaged from the bracket 130 with an upward motion. The container 100 with the exudate is discarded to medical waste. The housing 80 is engaged with a fresh container, in the manner described, and the wound which is to be treated is reconnected by means of the flexible hose 78 to the suction port 154 of the fresh container. The apparatus 10 inside the housing 80 is then made operative and the wound treatment process can continue.

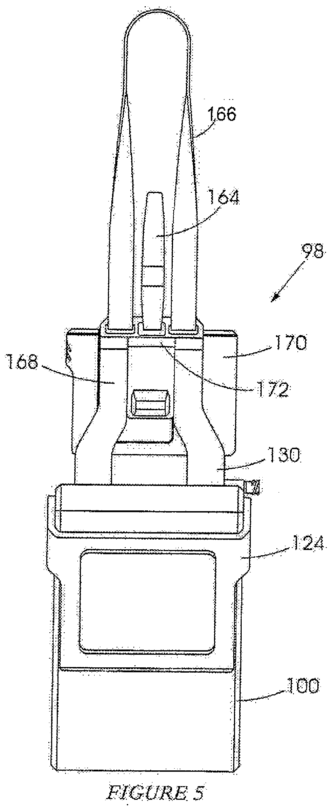

Figures 5, 6 and 7 are different views of the assembly 98 comprising the housing 80 and the container 100. In Figure 5 the handle 124 is moved to a position at which it faces a rear side of the housing.

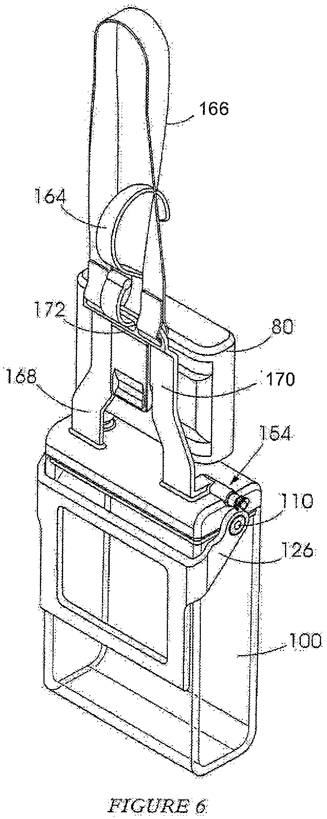

Figure 6 illustrates a hook 164 and a carry strap 166 which are attached to the bracket 130. The bracket has two legs 168 and 170 respectively with a bridge piece 172, between ends of the legs, which is configured so that it can be engaged with a snap action with the clip 84.

Figure 7 shows the assembly 98 from what may be regarded as a front side. It is apparent that the carry strap 166 or the hook 164 can be used, as required, either to carry the assembly 98 or to suspend the assembly from a suitable support.

The assembly 98 is lightweight and portable. Due to the compact configuration of the assembly the mobility of a user, who has a wound which is subjected to negative pressure wound therapy, is not unduly affected. The apparatus 10 operates in the manner described. User action is required to replace the container 100, when needed. This is easily done.

The information provided by the processor 22 via either the visual output 28 or the audible output 30 is such that a user can be prompted to take action to replace the container 100, to replace the batteries in the power source 20 and, more generally, to comply with such requirements which may be made available to the user as a result of details having previously been programmed into the processor 22.

## Claims

1. Apparatus (10) for controlling a negative pressure level prevailing at a wound site, the apparatus including an electric motor (42), a constant voltage source (24) which supplies current to the electric motor, an air pump (40) which is driven by the electric motor and which is configured to establish a negative pressure level in a region which encloses the wound site, a current monitor (26) which is configured to measure the amplitude of the current supplied to the motor while the pump is operable, a processor (22) which is configured to derive an operating current value of the motor which is dependent at least on said measured current amplitude, and a switch (56), responsive to the processor, to interrupt the supply of current to the motor when the operating current value equals a predetermined current value, **characterised in that** the apparatus includes a temperature sensor (48) which produces a signal, which is dependent on an operating temperature of the motor while the pump is operating, **in that** the processor (22) is operative, in response to said signal, to compensate for the effect of the operating temperature of the motor on the measured amplitude of the current supplied to the motor and **in that** the processor (22) is further operative to compensate for the effect of the no-load current of the motor on the measured amplitude of the current supplied to the motor, thereby to allow the processor to derive from the measured amplitude of the current supplied to the motor, an operating current value of the motor independently of the operating temperature of the motor and independently of the no-load current of the motor, whereby said derived operating current value determines said negative pressure value.

2. Apparatus according to claim 1 which includes a first housing (12A) in which the constant voltage source and the processor are mounted, a second housing (16A) in which the electric motor and the air pump are mounted, and a connector arrangement (60, 20, 62) which connects the first housing to the second housing.

3. Apparatus according to claim 1 which includes a housing (80) in which the constant voltage source (24), the processor (22), the electric motor (42) and the air pump (40) are mounted.

4. An assembly comprising the apparatus of claim 3, an exudate collecting container (100), with which the housing (80) is releasably engageable, the air pump (40) including an inlet which is in air communication with an interior (160) of the container, and a suction port (154) which, in use, is connected to the wound site and which is in air communication with the interior of the container.

5. An assembly according to claim 4 wherein the container (100) includes a lower part (102), an upper part (104) which is engageable with the lower part, an insert (114) at an interface between the lower part and the upper part whereby a manifold (150) is defined in the upper part, said manifold (150) being in air communication with an interior (160) of the lower part, a mechanism (124) which keeps the upper part engaged with the lower part, said inlet to the air pump being in air communication with an interior of the manifold, and said suction port being in air communication with the interior of the lower part.

6. An assembly according to claim 5 which includes an odour neutralizing filter (152) inside the manifold.

7. An assembly according to claim 5 wherein said insert (114) includes a plurality of air passages (148) which place the manifold (150) in air communication with the interior (160) of the lower part.

## Patentansprüche

1. Vorrichtung (10) zur Steuerung eines an einer Wundstelle herrschenden Unterdruckniveaus, wobei die Vorrichtung aufweist: einen Elektromotor (42), eine Konstantspannungsquelle (24), die den Elektromotor mit Strom versorgt, eine Luftpumpe (40), die vom Elektromotor angetrieben wird und dazu ausgelegt ist, ein Unterdruckniveau in einem die Wundstelle umgebenden Bereich zu erzeugen, einen Stromwächter (26), der so ausgelegt ist, dass er die Amplitude des dem Motor zugeführten Stroms misst, während die Pumpe in Betrieb ist, einen Prozessor (22), der so ausgelegt ist, dass er einen Betriebsstromwert des Motors ableitet, der zumindest von der gemessenen Stromamplitude abhängt, und einen Schalter (56), der auf den Prozessor anspricht, um die Stromzufuhr zum Motor zu unterbrechen, wenn der Betriebsstromwert einem vorgegebenen Stromwert entspricht, **dadurch gekennzeichnet, dass** die Vorrichtung einen Temperatursensor (48) aufweist, der ein Signal erzeugt, das von einer Betriebstemperatur des Motors abhängt, während die Pumpe in Betrieb ist, dass der Prozessor (22) so arbeitet, dass er als Reaktion auf das Signal den Einfluss der Betriebstemperatur des Motors auf die gemessene Amplitude des dem Motor zugeführten Stroms kompensiert, und dass der Prozessor (22) ferner so arbeitet, dass er den Einfluss des Leerlaufstroms des Motors auf die gemessene Amplitude des dem Motor zugeführten Stroms kompensiert, wodurch der Prozessor in die Lage versetzt wird, aus der gemessenen Amplitude des dem Motor zugeführten Stroms einen Betriebsstromwert des Motors unabhängig von der Betriebstemperatur des Motors und unabhängig vom Leerlaufstrom des Motors abzuleiten, wobei der abgeleitete Betriebsstromwert den Unterdruckwert bestimmt.

2. Vorrichtung nach Anspruch 1, die ein erstes Gehäuse (12A), in dem die Konstantspannungsquelle und der Prozessor untergebracht sind, ein zweites Gehäuse (16A), in dem der Elektromotor und die Luftpumpe untergebracht sind, sowie eine Verbindungsanordnung (60, 20, 62) aufweist, die das erste Gehäuse mit dem zweiten Gehäuse verbindet.

3. Vorrichtung nach Anspruch 1, die ein Gehäuse (80) aufweist, in dem die Konstantspannungsquelle (24), der Prozessor (22), der Elektromotor (42) und die Luftpumpe (40) untergebracht sind.

4. Anordnung, die die Vorrichtung nach Anspruch 3 sowie einen Exsudatsammelbehälter (100) aufweist, mit dem das Gehäuse (80) lösbar in Eingriff bringbar ist, wobei die Luftpumpe (40) einen Einlass aufweist, der in Luftverbindung mit einem Innenraum (160) des Behälters steht, sowie eine Ansaugöffnung (154), die im Betrieb mit der Wundstelle verbunden ist und die in Luftverbindung mit dem Innenraum des Behälters steht.

5. Anordnung nach Anspruch 4, wobei der Behälter (100) einen unteren Teil (102), einen oberen Teil (104), der mit dem unteren Teil in Eingriff gebracht werden kann, sowie einen Einsatz (114) an einer Schnittstelle zwischen dem unteren Teil und dem oberen Teil umfasst, wodurch im oberen Teil ein Verteiler (150) definiert wird, wobei der Verteiler (150) in Luftverbindung mit einem Innenraum (160) des Unterteils in Luftverbindung steht, einen Mechanismus (124), der den Oberteil mit dem Unterteil in Eingriff hält, wobei der Einlass zur Luftpumpe mit dem Innenraum des Verteilers in Luftverbindung steht und die Ansaugöffnung mit dem Innenraum des Unterteils in Luftverbindung steht.

6. Anordnung nach Anspruch 5, die einen Geruchsneutralisierungsfilter (152) innerhalb des Verteilers aufweist.

7. Anordnung nach Anspruch 5, wobei der Einsatz (114) eine Vielzahl von Luftkanälen (148) aufweist, die den Verteiler (150) mit dem Innenraum (160) des Unterteils in Luftverbindung bringen.

## Revendications

1. Appareil (10) destiné à réguler un niveau de pression négative régnant au niveau d'un site de plaie, l'appareil comprenant un moteur électrique (42), une source de tension constante (24) qui alimente le moteur électrique en courant, une pompe à air (40) entraînée par le moteur électrique et configurée pour établir un niveau de pression négative dans une zone qui entoure le site de plaie, un moniteur de courant (26) configuré pour mesurer l'amplitude du courant fourni au moteur pendant que la pompe est en fonctionnement, un processeur (22) configuré pour dériver une valeur de courant de fonctionnement du moteur qui dépend au moins de ladite amplitude de courant mesurée, et un commutateur (56), réagissant au processeur, pour interrompre l'alimentation en courant du moteur lorsque la valeur de courant de fonctionnement est égale à une valeur de courant prédéterminée, **caractérisé en ce que** l'appareil comprend un capteur de température (48) qui produit un signal dépendant de la température de fonctionnement du moteur pendant que la pompe fonctionne, **en ce que** le processeur (22) est conçu pour, en réponse audit signal, compenser l'effet de la température de fonctionnement du moteur sur l'amplitude mesurée du courant fourni au moteur, et **en ce que** le processeur (22) est en outre conçu pour compenser l'effet du courant à vide du moteur sur l'amplitude mesurée du courant fourni au moteur, permettant ainsi au processeur de déduire, à partir de l'amplitude mesurée du courant fourni au moteur, une valeur de courant de fonctionnement du moteur indépendamment de la température de fonctionnement du moteur et indépendamment du courant à vide du moteur, ladite valeur de courant de fonctionnement déduite déterminant ladite valeur de pression négative.

2. Appareil selon la revendication 1, qui comprend un premier boîtier (12A) dans lequel sont montés la source de tension constante et le processeur, un deuxième boîtier (16A) dans lequel sont montés le moteur électrique et la pompe à air, et un agencement de connecteurs (60, 20, 62) qui relie le premier boîtier au deuxième boîtier.

3. Appareil selon la revendication 1, qui comprend un boîtier (80) dans lequel sont montés la source de tension constante (24), le processeur (22), le moteur électrique (42) et la pompe à air (40).

4. Ensemble comprenant l'appareil de la revendication 3, un récipient de collecte d'exsudat (100), avec lequel le boîtier (80) peut s'engager de manière amovible, la pompe à air (40) comprenant une entrée qui est en communication d'air avec un intérieur (160) du récipient, et un orifice d'aspiration (154) qui, en utilisation, est relié au site de la plaie et qui est en communication d'air avec l'intérieur du récipient.

5. Ensemble selon la revendication 4, dans lequel le récipient (100) comprend une partie inférieure (102), une partie supérieure (104) pouvant s'engager avec la partie inférieure, un insert (114) situé à l'interface entre la partie inférieure et la partie supérieure, de sorte qu'un collecteur (150) est défini dans la partie supérieure, ledit collecteur (150) étant en communication d'air avec l'intérieur (160) de la partie inférieure, un mécanisme (124) qui maintient la partie supérieure en prise avec la partie inférieure, ladite entrée de la pompe à air étant en communication d'air avec l'intérieur du collecteur, et ledit orifice d'aspiration étant en communication d'air avec l'intérieur de la partie inférieure.

6. Ensemble selon la revendication 5, qui comprend un filtre neutralisant les odeurs (152) à l'intérieur du collecteur.

7. Ensemble selon la revendication 5, dans lequel ledit insert (114) comprend une pluralité de passages d'air (148) qui mettent le collecteur (150) en communication d'air avec l'intérieur (160) de la partie inférieure.
